(19) Europäisches Patentamt
European Patent Office
Office européen des brevets

(11) Publication number : 0 450 812 B1

(12) EUROPEAN PATENT SPECIFICATION

(45) Date of publication of patent specification : 27.04.94 Bulletin 94/17

(51) Int. Cl.$^5$ : C07D 493/08, A61K 31/335, A01N 43/24, C12P 17/04

(21) Application number : 91302458.4

(22) Date of filing : 21.03.91

# (54) Antihypercholesterolemics.

(30) Priority : 21.03.90 US 496742
21.03.90 US 496731
21.03.90 US 496749
11.12.90 US 626071

(43) Date of publication of application : 09.10.91 Bulletin 91/41

(45) Publication of the grant of the patent : 27.04.94 Bulletin 94/17

(84) Designated Contracting States : AT BE CH DE DK ES FR GB GR IT LI LU NL SE

(56) References cited : US-A- 4 276 216

(73) Proprietor : MERCK & CO. INC.
126, East Lincoln Avenue P.O. Box 2000
Rahway New Jersey 07065-0900 (US)

(72) Inventor : Bergstrom, James D.
60 Rohill Road
Neshanic, NJ 08853 (US)
Inventor : Liesch, Jerrold M.
10 Sherbrooke Drive
Princeton Junction, NJ 08550 (US)
Inventor : Hensens, Otto D.
65 Alexander Drive, River Plaza
Red Bank, NJ 07701 (US)
Inventor : Onishi, Janet C.
350 Cherry Hill Road
Mountainside, NJ 07092 (US)
Inventor : Huang, Leeyuan
33 Will Lane
Watchung, NJ 07060 (US)
Inventor : Vanmiddlesworth, Frank L.
199 Second Street
Fanwood, NJ 07023 (US)
Inventor : Diez, Maria T.
C/Olid, 4
E-28010 Madrid (ES)
Inventor : Bartizal, Kenneth F.
16 Lilac Lane
Somerset, NJ 08873 (US)
Inventor : Nallin, Mary
634 Westfield Avenue
Westfield, NJ 07090 (US)
Inventor : Rozdilsky, Walter
949 North Concourse
Cliffwood Beach, NJ 07735 (US)
Inventor : Perez, Fernando Pelaez
C/Camino Valderribas
n-110,4- C-28038 Madrid (ES)

(74) Representative : Thompson, John Dr. et al
Merck & Co., Inc. European Patent
Department Terlings Park Eastwick Road
Harlow, Essex CM20 2QR (GB)

EP 0 450 812 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

BACKGROUND OF THE INVENTION

Hypercholesterolemia is known to be one of the prime risk factors for ischemic cardiovascular disease, such as arteriosclerosis. Bile acid sequestrants have been used to treat this condition; they seem to be moderately effective but they must be consumed in large quantities, i.e. several grams at a time, and they are not very palatable.

MEVACOR® (lovastatin), now commercially available, is one of a group of very active antihypercholesterolemic agents that function by limiting cholesterol biosynthesis by inhibiting the enzyme, HMG-CoA reductase.

Squalene synthetase is the enzyme involved in the first committed step of the de novo cholesterol biosynthetic pathway. This enzyme catalyzes the reductive dimerization of two molecules of farnesyl pyrophosphate to form squalene. The inhibition of this committed step to cholesterol should leave unhindered biosynthetic pathways to ubiquinone, dolichol and isopentenyl t-RNA.

Previous efforts at inhibiting squalene synthetase have employed pyrophosphate or pyrophosphate analog containing compounds such as those described in P. Ortiz de Montellano et al, J. Med Chem. 20, 243 (1977) and E.J. Corey and R. Volante, J. Am. Chem. Soc., 98, 1291 (1976). S. Biller (U.S. Patent 4,871,721) describes isoprenoid (phosphinylmethyl)phosphonates as inhibitors of squalene synthetase.

The present invention provides nonphosphorus containing inhibitors of squalene synthetase.

DETAILED DESCRIPTION OF THE INVENTION

The present invention is directed to novel compounds of structural formula (I) which are squalene synthetase inhibitors:

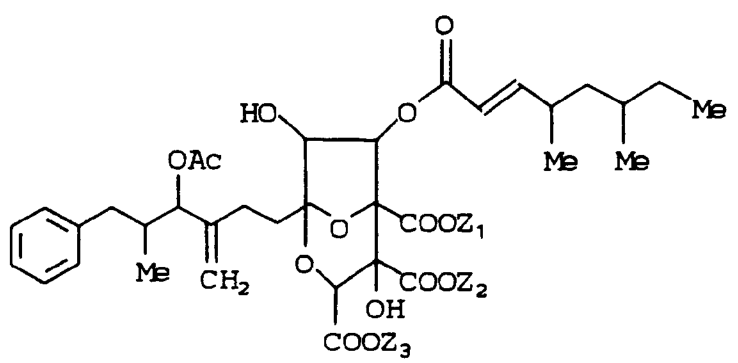

(I)

wherein

$Z_1$, $Z_2$ and $Z_3$ are each independently selected from;

a) H;

b) $C_{1-5}$alkyl;

c) $C_{1-5}$alkyl substituted with a member of the group consisting of:

i) phenyl,

ii) phenyl substituted with methyl, methoxy, halogen (Cl, Br, I, F) or hydroxy; or

a pharmaceutically acceptable salt of a compound of of formula (I) in which at least one of $Z_1$, $Z_2$ and $Z_3$ is hydrogen.

In one embodiment of the present invention are those compounds of formula (I) wherein the relative stereochemical configuration of the 2,8-dioxabicyclo[3.2.1]octane ring is as shown below:

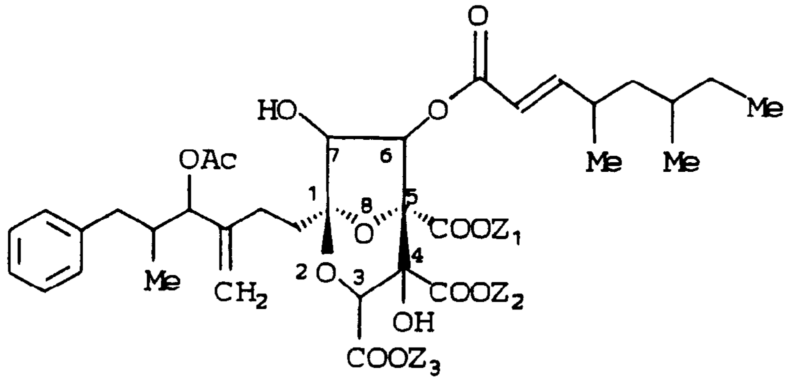

(I)

Throughout this specification and claims where stereochemistry is described for the dioxabicyclo[3.2.1]-octane ring the configuration implied is relative. The actual configuration may be as shown or that of its enantiomer.

Further illustrating this embodiment are those compounds of structural formula (I) wherein the relative configuration at positions 3, 6 and 7 is as shown below:

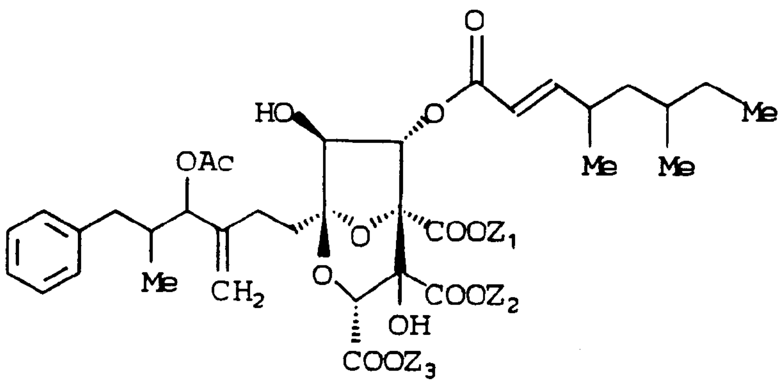

(I)

In one class of this embodiment are those compounds of structure (I) wherein the relative configuration at the 4-position is as shown below:

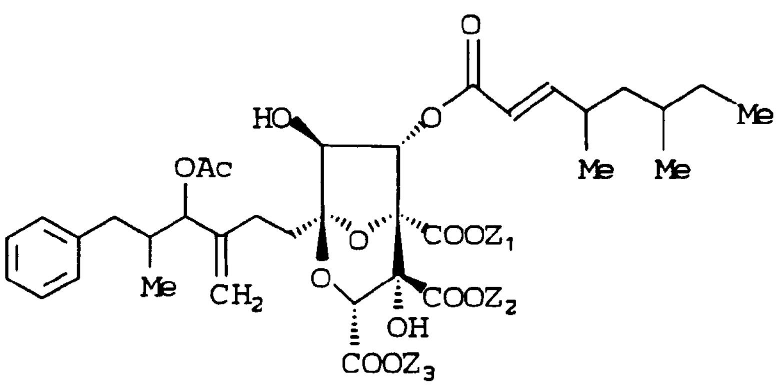

(I)

Exemplifying this class is the compound wherein $Z_1$, $Z_2$ and $Z_3$ are each hydrogen or a pharmaceutically

acceptable salt thereof. The compound wherein $Z_1$, $Z_2$ and $Z_3$ are each hydrogen is hereafter referred to as compound A.

Further illustrating this class are those compounds in which one or more of $Z_1$, $Z_2$ or $Z_3$ is $C_{1-5}$alkyl or $C_{1-5}$alkyl substituted with phenyl or phenyl substituted with methyl, methoxy, halogen (Cl, Be, I, F) or hydroxy. In a specific illustration, $Z_1$, $Z_2$ and $Z_3$ are each methyl. This compound is hereafter referred to as compound B.

The compounds of formula (I) are prepared in an aerobic fermentation procedure employing a novel culture, MF5453, observed as a sterile mycelium. Mutants of MF5453 are also capable of producing compounds of this invention and are included within this invention.

The culture MF5453 is that of a fungus isolated from a water sample obtained from the Jalon river, Zaragoza, Spain. This culture has been deposited with the American Type Culture Collection at 12301 Parklawn Drive, Rockville, MD 20852 as ATCC 20986.

The microorganism MF5453 exhibits the following morphological characteristics:

Colonies 22-24 mm in diameter in two weeks on potato-dextrose agar (Difco) at 25°C in continuous fluorescent light, 40-45 mm diameter in two weeks on deer dung-extract agar (Mycology Guidebook, media M-11, p. 660) at 25°C. Colonies on potato-dextrose agar consisting of moderately thick, 1-2 mm deep, mycelium, cottony at the center, becoming felty to velutinous towards the margin, forming obscure concentric ridges, occasionally forming sectors of differing mycelial texture and color, margin entire, without a fringe of submerged leading hyphae. Colony color at first white or some white aerial hyphae, soon becoming cream, Cream Color, Cartridge Buff, (capitalized color names from Ridgway, R. Color Standards and Nomenclature, Washington, D.C. 1912), finally light gray to gray, Light Olive-Gray, Gray, Pallid Neutral Gray, Neutral Gray, Gull Gray, Deep Gull Gray. Colony reverse grayish brown to yellow brown or cream, Cinnamon-Drab, Cinnamon, Ochraceous-Buff, Antimony Yellow, Warm Buff, Cartridge Buff. Odors and exudates absent.

Hyphae ascomycetous in morphology, undifferentiated, septate, branched 1.5-3.5 µm in diameter, hyaline in water and KOH. No reproductive structures formed on any of cultural conditions surveyed, including incubation in both continuous light and 12/12 hour dark cycles on either cornmeal agar, hay extract agar, oatmeal agar, dung extract agar, and malt extract agar.

Compounds of this invention can be obtained by culturing the above noted microorganism in an aqueous nutrient medium containing sources of assimilable carbon and nitrogen, preferably under oaerobic conditions. Nutrient media may also contain mineral salts and defoaming agents.

The present invention thus provides a process of making a compound of structure:

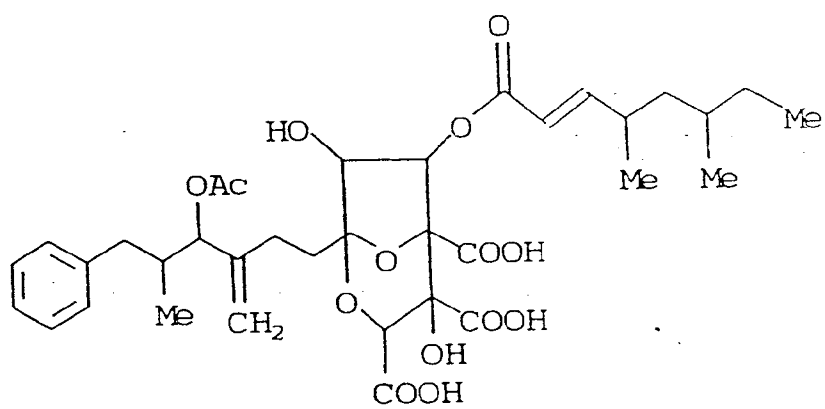

comprising cultivating MF5453 (ATCC 20986), or an active mutant thereof, in an aqueous nutrient medium containing sources of assimilable nitrogen and carbon and recovering the compound.

The preferred sources of carbon in the nutrient medium are carbohydrates such as glucose, glycerin, starch, dextrin, and the like. Other sources which may be included are maltose, mannose, sucrose, and the like. In addition, complex nutrient sources such as oat flour, corn meal, millet, corn and the like may supply utilizable carbon. The exact quantity of the carbon source which is used in the medium will depend, in part, upon the other ingredients in the medium, but is usually found in an amount ranging between 0.5 and 5 percent by weight. These carbon sources can be used individually in a given medium or several sources in combination in the same medium.

The preferred sources of nitrogen are amino acids such as glycine, methionine, proline, threonine and the like, as well as complex sources such as yeast extracts (hydrolysates, autolysates), dried yeast, tomato paste, soybean meal, peptone, corn steep liquor, distillers solubles, malt extracts and the like. Inorganic nitrogen

sources such as ammonium salts (eg. ammonium nitrate, ammonium sulfate, ammonium phosphate, etc.) can also be used. The various sources of nitrogen can be used alone or in combination in amounts ranging between 0.2 to 90 percent by weight of the medium.

The carbon and nitrogen sources are generally employed in combination, but need not be in pure form. Less pure materials which contain traces of growth factors, vitamins, and mineral nutrients may also be used. Mineral salts may also be added to the medium such as (but not limited to) calcium carbonate, sodium or potassium phosphate, sodium or potassium chloride, magnesium salts, copper salts, cobalt salt and the like. Also included are trace metals such as manganese, iron, molybdenum, zinc, and the like. In addition, if necessary, a defoaming agent such as polyethylene glycol or silicone may be added, especially if the culture medium foams seriously.

The preferred process for production of compounds of this invention consists of inoculating spores or mycelia of the producing organism into a suitable medium and then cultivating under aerobic condition.

The fermentation procedure generally is to first inoculate a preserved source of culture into a nutrient seed medium and to obtain, sometimes through a two step process, growth of the organisms which serve as seeds in the production of the active compounds. After inoculation, the flasks are incubated with agitation at temperatures ranging from 20 to 30°C, preferably 25 to 28°C. Agitation rates may range up to 400 rpm, preferably 200 to 220 rpm. Seed flasks are incubated over a period of 2 to 10 days, preferably 2 to 4 days. When growth is plentiful, usually 2 to 4 days, the culture may be used to inoculate production medium flasks. A second stage seed growth may be employed, particularly when going into larger vessels. When this is done, a portion of the culture growth is used to inoculate a second seed flask incubated under similar condition but employing shorter time.

After inoculation, the fermentation production medium is incubated for 3 to 30 days, preferably 4 to 14 days, with or without agitation (depending on whether liquid or solid fermentation media are employed). The fermentation is conducted aerobically at temperatures ranging from 20 to 40°C. If used, agitation may be at a rate of 200 to 400 rpm. To obtain optimum results, the temperatures are in the range of 22 to 28°C, most preferably 24 to 26°C. The pH of the nutrient medium suitable for producing the active compounds is in the range of 3.55 to 8.5, most preferably 5.0 to 7.5. After the appropriate period for production of the desired compound, fermentation flasks are harvested and the active compound isolated.

The pH of the aqueous mycelial fermentation is adjusted to between 1 and 9 (preferably between 3 and 5) preferably mixed with a water miscible solvent such as 50% methanol and the mycelia filtered. The active compound may then be isolated from the aqueous filtrate by several methods including:

1. Liquid-liquid extraction of the aqueous filtrate into a water immiscible solvent such as methyl elthyl ketone, ethyl acetate, diethyl ether, or dichloromethane preferably after having adjusted the pH to between 3 and 5.
2. Solid-liquid extraction of the aqueous filtrate onto an organic matrix such as SP207 or HP-20 and elution with an organic solvent (aqueous or non aqueous) such as 90/10 methanol/water or 90/10 acetone/water.
3. Adsorption of the active compound from the aqueous filtrate onto an ionic exchange resin such as Dowex 1($Cl^-$) or Dowex 50 ($Ca^{2+}$) and elution with a high ionic strength organic/aqueous solvent such as 90/10 methanol/aqueous 30% $NH_4Cl$. This material could then be desalted by employing either method 1 or 2 above.

Each of these three methods may also be used in the further purification of the active compound.

The fraction containing active compound from the above methods could then be dried in vacuo leaving the crude active compound. The crude active compound is then generally subjected to several separation steps such as adsorption and partition chromatography, and precipitation. For each separation step, fractions are collected and combined based on results from an assay and/or HPLC analysis.

The chromatographic separations may be carried out by employing conventional column chromatography with ionic or nonionic adsorbent. When silica gel is the adsorbent, an alcohol/chlorohydrocarbon/organic acid mixture such as methanol/chloroform/acetic acid/ water is useful as an eluant. For reverse phase chromatography, the preferred adsorbent is a C8 bonded phase silica gel. The preferred eluant for reverse phase chromatography is a mixture of acetonitrile and water buffered at a low pH, such as 0.1% phosphoric acid, or trifluoroacetic acid. The active compound can be precipitated out of a non-polar solvent as the quinine salt. The preferred solvent for precipitation is diethyl ether.

The present invention is also directed to a method of inhibiting cholesterol biosynthesis which comprises the administration to a subject in need of such treatment a nontoxic therapeutically effective amount of a compound represented by structural formula (I) and pharmaceutically acceptable salts thereof. Specifically the compounds of this invention are useful as antihypercholesterolemic agents for the treatment of arteriosclerosis, hyperlipidemia, familial hypercholesterolemia and the like diseases in humans.

The present invention thus provides a compound of formula (I), or a pharmaceutically acceptable salt

thereof for the manufacture of a medicament for treating hypercholesterolemia.

Compounds of the invention may be administered orally or parenterally in the form of a capsule, a tablet, an injectable preparation or the like. It is usually desirable to use the oral route. Doses may be varied, depending on the age, severity, body weight and other conditions of human patients but daily dosage for adults is within a range of from about 20 mg to 2000 mg (preferably 20 to 100 mg) which may be given in two to four divided doses. Higher doses may be favorably employed as required.

The pharmaceutically acceptable salts of the compounds of this invention include those formed from cations such as sodium, potassium, aluminum, calcium, lithium, magnesium, zinc, and from bases such as ammonia, ethylenediamine, N-methyl-glucamine, lysine, arginine, ornithine, choline, N,N'-dibenzylethylenediamine, chloroprocaine, diethanolamine, procaine, N-benzylphenethylamine, diethylamine, piperazine, tris(hydroxymethyl)aminomethane, and tetramethylammonium hydroxide. This invention includes salts of one, two or three of the carboxyl groups of formula (I).

The compounds of this invention may also be coadministered with pharmaceutically acceptable nontoxic cationic polymers capable of binding bile acids in a non-reabsorbable form in the gastro-intestinal tract. Examples of such polymers include cholestyramine, colestipol and poly[methyl-(3-tri-methylaminopropyl)iminotrimethylene dihalide]. The relative amounts of the compounds of this invention and these polymers is between 1:100 and 1:15,000.

The intrinsic squalene synthetase inhibitory activity of representative compounds of this invention was measured by the standard in vitro protocol described below:

Preparation of Microsomes:

Male, Charles River CD rats (120 to 150 g) were fed a diet containing 0.1% lovastatin for 4 days. The livers from these rats were homogenized in 5 volumes (ml/g) of ice cold 50 mM HEPES (4-(2-hydroxyethyl)-1-piperazine-ethanesulfonic acid), 5 mM EDTA(ethylenediaminetetraacetic acid) pH 7.5 with a Potter-Elvehjem type tissue grinder. The homogenate was centrifuged twice at 20,000 x g for 15 minutes at 4°C, discarding the pellet each time. The supernatant was then centrifuged at 100,000 x g for 1 hour at 4°C. The resulting microsomal pellet was resuspended in a volume of the above homogenizing buffer equal to one-fifth the volume of the original homogenate. This microsomal preparation has a protein concentration of about 7 mg/ml. The microsomal suspensions were stored in aliquots at -70°C. Squalene synthetase activity in these aliquots is stable for at least several months.

Partial Purification of Prenyl Transferase

Prenyl transferase was purified to use in the enzymatic synthesis of radiolabelled farnesyl pyrophosphate. Prenyl transferase was assayed by the method of Rilling (Methods in Enzymology 110, 125-129 (1985)) and a unit of activity is defined as the amount of enzyme that will produce 1 μmole of farnesyl pyrophosphate per minute at 30°C in the standard assay.

The livers of 23 forty-day old male rats that had been fed 5% cholestyramine plus 0.1% lovastatin were homogenzied in a Waring blender in 1 liter of 10 mM mercaptoethanol, 2 mM EDTA, 25 μM leupeptin, 0.005% phenylmethyl sulfonyl fluoride pH 7.0 containing 0.1 trypsin inhibitor units of aprotinin/ml. The homogenate was centrifuged at 20,000 x g for 20 minutes. The supernatant was adjusted to pH 5.5. with 6N HOAc and centrifuged at 100,000 x g for 1 hour. This supernatant was adjusted to pH 7.0 with 3N KOH and a 35-60% ammonium sulfate fraction taken. The 60% pellet was redissolved in 60 ml of 10 mM potassium phosphate, 10 mM mercaptoethanol, 1 mM EDTA pH 7.0 (Buffer A) and dialyzed against two 1 liter changes of Buffer A. This dialyzed fraction was applied to a 12.5 x 5 cm column of DEAE-sepharose 4B equilibrated with Buffer A. The column was washed with 700 ml of Buffer A and a 1 liter gradient from Buffer A to 100 mM potassium phosphate, 10 mM mercaptoethanol, 1 mM EDTA pH 7.0. Fractions having a specific activity greater than 0.20 units/mg were combined, solid ammounium sulfate was added to bring to 60% saturation and pelleted. The pellet was dissolved in 8 ml of 10 mM Tris, 10 mM β-mercaptoethanol pH 7.0 (Buffer B). The redissolved pellet was taken to 60% saturation with ammonium sulfate by adding 1.5 volumes of saturated ammonium sulfate in Buffer B. This ammonium sulfate suspension contained 3.5 units/ml with a specific activity of 0.23 units/mg and was free of isopentenyl pyrophosphate isomerase activity. This ammonium sulfate suspension was used for the synthesis of [4-$^{14}$C]farnesyl-pyrophosphate and its activity was stable stored at 4°C for at least 6 months.

Enzymatic Synthesis of [4-$^{14}$C]farnesyl-pyrophosphate

The solvent (ethanol: 0.15 $NH_4OH$, 1:1) was removed from 55 μCi of [4-$^{14}$C]isopentenyl pyrophosphate

(47.9 μCi/μmole) by rotary evaporation. Six hundred microliters of 100 mM Tris, 10 mM $MgCl_2$, 4 mM dithiothreitol pH 7.5 was added and the solution was transferred to a 1.5 ml Eppendorf centrifuge tube. Geranylpyrophosphate, 250 μl of a 20 mM solution, and 50 μl of the ammonium sulfate suspension of prenyl transferase were added to initiate the reaction. This incubation contained 5 μmoles of geranyl pyrophosphate, 1.15 μmoles of isopentenyl pyrophosphate, 6 μmoles of $MgCl_2$ and 0.18 units of prenyl transferase in a volume of 900 μl. The incubation was conducted at 37°C. During the incubation, the mix turned cloudy white as the newly formed magnesium complex of farnesyl pyrophoshate precipitated out of solution. The [4-$^{14}$C]farnesyl pyrophosphate was collected by centrifugation for 3 minutes at 14,000 rpm in an Eppendorf centrifuge tube, the supernatant removed, and the pellet was dissolved in 1.0 ml of 50 mM HEPES, 5 mM EDTA, pH 7.5 The yield was 50.7 μCi (92%) of [4-$^{14}$C]farnesyl pyrophosphate.

The [4-$^{14}$C]farnesyl pyrophosphate was stored in aliquots at -70°C.

Squalene Synthetase Assay

Reaction were performed in 16 x 125 mm screw cap test tubes. A batch assay mix was prepared from the following solution:

| | | μl per assay | volume for 50 assays |
|---|---|---|---|
| 1. | 250 mM Hepes pH 7.5 | 20 | 1000 |
| 2. | NaF 110 mM | 10 | 500 |
| 3. | $MgCl_2$ 55 mM | 10 | 500 |
| 4. | Dithiothreitol 30 mM | 10 | 500 |
| 5. | NADPH 10 mM (made fresh) | 10 | 500 |
| 6. | [4-$^{14}$C]farnesyl-pyrophosphate 47.9 μCi/μmole, and 0.025 μCi/3.0 μl | 3.0 | 150 |
| 7. | $H_2O$ | 24 | 1200 |

This assay mix was degassed under a vacuum and flushed with $N_2$. Solutions of the squalene synthetase inhibitors were prepared either in DMSO or MeOH and a 1:120 dilution of the microsomal protein was made with the original homogenizing buffer. For each reaction, 87 μl of the assay mix was taken with 3 μl of an inhibitor solution (DMSO or MeOH in the controls), warmed to 30°C in a water bath and then the reaction was initiated by the additon of 10 μl of the 1:120 dilution of microsomal protein (0.6 μg protein total in the assay). The reactions were stopped after 20 minutes by the addition of 100 μl of a 1:1 mix of 40% KOH with 95% EtOH. The stopped mix was heated at 65°C for 30 minutes, cooled, 10 ml of heptane was added and the mix was vortexed. Two g of activated alumina was then added, the mix vortexed again, the alumina allowed to settle and 5 ml of the heptane layer was removed. Ten ml of scintillation fluid was added to the heptane solution and radioactivity was determined by liquid scintillation counting.

Percent inhibition is calulated by the formula:

$$1 - \frac{[\text{Sample} - \text{Blank}]}{[\text{Control} - \text{Blank}]} \times 100$$

$IC_{50}$ values were determined by plotting the log of the concentration of the test compound versus the percentage inhibition. The $IC_{50}$ is the concentration of inhibitor that give 50% inhibition as determined from these plots.

Representative of the intrinsic squalene synthetase inhibitory activities of the compounds of this invention are the $IC_{50}$ data tabulated below:

| Compound | Squalene Synthetase $IC_{50}$ |
|---|---|
| Compound A | ≤ 5 μM |
| Compound B | ≤ 5 μM |

The present compounds also demonstrate broad spectrum antifungal activity as determined by broth or agar dilution methods. The compounds are particularly active towards filamentous fungi and yeasts including Candida albicans and Crypt neoformans. The sensitivity of filamentous fungi and yeast was determined using inhibitor dilution assays in microtiter format. The compounds were dissolved in DMSO at 2 mg/ml and serially diluted in 0.1 M phosphate buffer, pH 7.0 in the microtiter dish from 100 to 0.006 μg/ml. A standardized spore suspension for testing the filamentous fungi was prepared by inoculating Antibiotic Medium #3 containing 1.5% agar with spores such that 1.5 x $10^3$ colony forming units were added per well. The microtiter wells were filled with 50 μl of buffer containing compound and 50 μl of inoculated medium. The sensitivity of yeasts was determined by inoculating yeast nitrogen base containing 1% dextrose (YNB/G) with aliquots of an overnight yeast culture grown in yeast morphology (YM) media at 35°C and diluting in YNB/G to yield a final concentration of 1.5-7.5 x $10^3$ colony forming units/well. One hundred fifty μl of inoculated media was added per well. To test the sensitivity of yeast, compound was solubilized in 10 percent aqueous DMSO at 2.56 mg/ml. The compound was diluted serially in YNG/G from 128 to 0.06 μg/ml. The wells were filled with 150 μl of drug containing media. The minimum inhibitory concentration (MIC) is defined as the lowest concentration to prevent visible growth after an incubation for 42 hours, 28°C for the filamentous fungi and 24 hours, 28°C for the yeasts. Minimum fungicidal concentration in μg/ml is defined as the lowest concentration of drug that totally prevented growth or permitted growth of no more than three colonies. Representative of the antifungal activity are the minimum inhibitory concentration and minimum fungicidal concentration data shown below:

Minimum Inhibitory Concentration (mcg/ml)

| Organism Filamentous Fungi | Compound A | Compound B |
|---|---|---|
| A. fumigatus MF4839 | 6.2 | >128 |
| A. flavus MF383 | 6.2 | >128 |
| A. niger MF442 | 25 | - |
| Fus. oxysporum MF4014 | >100 | - |
| Pen. italicum MF2819 | 6.2 | - |
| Coch. miyabeanus MF4626 | 1.6 | - |
| T. Mentagrophytes MF4864 | 4 | >128 |

| Yeast | Compound A | Compound B |
|---|---|---|
| C. albicans MY1055 | 8 | >128 |
| C. tropicalis MY1012 | 4 | >128 |
| C. parapsilosis MY1010 | 8 | >128 |
| Crypt. neoformans MY1051 | 1 | >128 |

Minimum Fungicidal Concentration (μg/ml)

| | Compound A | Compound B |
|---|---|---|
| Yeast | | |
| C1 albicans MY1055 | 4 | >128 |
| C1 tropicalis MY1012 | 2 | >128 |
| C1 parapsilosis MY1010 | 2 | >128 |
| Crypt neoformans MY1051 | 0.5 | >128 |

Based on the above MIC and MFC data it is determined that generally from 2 to 5 mg/kg should be employed as a unit dosage in an antifungal treatment.

The present invention thus provides a method for inhibiting fungal growth in soil or plant parts, or in inanimate objects, comprising applying to the area where growth is to be controlled an antifungally effective amount of a compound of formula (I).

The present invention also provides the use of a compound of formula (I) for the manufacture of a medicament suitable for inhibiting fungal growth in a living organism.

The compounds of this invention are adaptable to being utilized in various applications of antifungal compositions. In such use, compounds may be admixed with a biologically inert carrier, generally with the aid of a surface active dispersing agent, the nature of which would vary depending on whether the use is for the control of pathogens infecting mammals, such as man, or birds, or reptiles, or for control of fungi in agriculture such as in soil or plant parts, or for the control of fungi in inanimate objects.

In compositions for medical applications, the compounds may be admixed with a pharmaceutically acceptable carrier, the nature of which will vary depending on whether the composition is to be topical, parenteral or oral.

The present invention provides a pharmaceutical composition comprising a nontoxic therapeutically effective amount of a compound of formula (I) and a pharmaceutically acceptable carrier.

If application is to be topical, the drug may be formulated in conventional creams and ointments such as white petroleum, anhydrous lanolin, cetyl alcohol, cold cream, glyceryl monostearate, rose water and the like.

For parenteral applications, the compounds may be formulated in conventional parenteral solutions such as 0.85 percent sodium chloride or 5 percent dextrose in water, or other pharmaceutically acceptable compositions.

Compositions for oral administration may be prepared by intimately mixing the component drugs with any of the usual pharmaceutical media, including, for liquid preparations, liquid carriers kaolin, ethyl cellulose, surface active dispersing agents, generally with lubricant such as calcium stearate, together with binders, disintegrating agents and the like.

These compositions are then administered in amounts sufficient to obtain the desired antifungal effect. For medical application, the method comprises administering to a subject in need of treatment a therapeutically effective antifungal amount of a compound of Formula I. The appropriate doses will vary depending on age, severity, body weight and other conditions. For topical application the compositions are applied directly to the area where control is desired. For internal administration, the composition may be applied by injection or may be administered orally.

For non-medical application, the product of the present invention, either singly or as a mixture, may be employed in compositions in an inert-carrier which includes finely divided dry or liquid diluents, extenders, fillers, conditioners and excipients, including various clays, diatomaceous earth, talc, and the like, or water and various organic liquids such a lower alkanols, for example ethanol and isopropanol, or kerosene, benzene, toluene and other petroleum distillate fractions or mixtures thereof.

These compositions may be employed by applying to the surface of or incorporating in the medium to be protected. For the control of rice blast, tomato late blight, tomato early blight, wheat leaf rust, bean powdery mildew and tomato Fusarium wilt, the compositions may be applied directly to the plant in topical application or administered to the soil for systemic application. The method comprises administering to the affected plant, soil or medium to be protected an antifungally effective amount of the compound of Formula I.

The following examples illustrate the preparation of the compounds of formula (I) and their incorporation into pharmaceutical compositions and as such are not to be considered as limiting the invention set forth in the claims appended hereto. The preferred route to Compound A is Example 2.

The composition of media employed in the following Examples are listed below:

| KF SEED MEDIUM | per liter |
|---|---|
| Corn Steep Liquor | 5 g |
| Tomato Paste | 40 g |
| Oat Flour | 10 g |
| Glucose | 10 g |
| Trace Element Mix | 10 ml |

pH adjusted to 6.8 (presterile)
50 mls/nonbaffled 250 mls Erlenmeyer flask
autoclave 20 minutes (121°C, 15 psi)

| Trace Elements Mix | g/L |
|---|---|
| $FeSO_4 \bullet 7H_2O$ | 1.0 |
| $MnSO_4 \bullet 4H_2O$ | 1.0 |
| $CuCl_2 \bullet 2H_2O$ | 0.025 |
| $CaCl_2 \bullet 2H_2O$ | 0.1 |
| $H_3BO_3$ | 0.056 |
| $(NH_4)_6Mo_7O_{24} \bullet 4H_2O$ | 0.019 |
| $ZnSO_4 \bullet 7H_2O$ | 0.2 |

dissolved in 1L 0.6 N HCl

| MBM Production Medium | g/L |
|---|---|
| Malt extract (Difco) | 5.0 |
| Glucose | 15.0 |
| Peptone | 1.0 |
| $KH_2PO_4$ | 1.0 |
| $MgSO_4$ | 0.5 |
| distilled $H_2O$ | 1000.0 mls |

(no pH adjustment)
45 mls/nonbaffled 250 mls Erlenmeyer flask autoclave 15 minutes (121°C, 15 psi)

EXAMPLE 1

Preparation of Compound A

A. Culturing MF5453

Culture MF5453 was inoculated into KF seed medium using one glass scoop of the original soil tube. The KF seed flask was incubated for 73 hours at 25°C, 220 rpm, 85% humidity. At the end of this incubation, 2.0 mls aliquots were aseptically transferred to each of 75 MBM production medium flasks. These production flasks were then incubated at 25°C, 220 rpm, 85% humidity, with a fermentation cycle of 14 days. Flasks were harvested as follows: mycelial growth was homogenized for 20 seconds at high speed using a Biohomogenizer/mixer (Biospec Products Inc. Bartlesville, Ok); and then 45 mls methanol was added to each flask (final methanol concentration was approximately 50%). Flasks were then returned to the shaker and agitated at 220 rpm for 30 minutes. Subsequently, the contents of the flasks were pooled.

B. Isolation of Compound A

A 6 liter 50% methanol homogenized fungal extract exhibiting a pH of 4.5 was employed in the following isolation procedure. The mycelia was filtered through celite and the recovered mycelia was extracted again by stirring overnight with 3 L of 50% methanol and again filtered.

The combined extract (9 L) of 50% methanol was diluted to 25% methanol with water (total volume 18 L) and applied to a Mitsubishi HP-20 column (750 ml) at a flow rate of 80 ml/minute. The column was washed with water (1 L) and eluted with a stepwise gradient of methanol consisting of 50/50 methanol/$H_2O$ (1 L), 60/40, methanol/$H_2O$ (1 L), 80/20 methanol/$H_2O$ (2 L,) 90/10 methanol/$H_2O$ (1 L), 100% methanol (2 L), and 100% acetone (1 L). The fractions from 50/50 to 90/10 methanol/$H_2O$ were combined and diluted with water to 35/65 methanol/$H_2O$ (total volume 10 L).

The 10 L of 35/65 methanol/$H_2O$ was acidified with 1.0 N HCl (20 ml) to pH 3.0 and extracted into EtOAc (4 L). The EtOAc layer was separated and the solvent removed in vacuo to yield 260 mg of an orange oil.

A portion (10%) of the orange oil was dissolved in 1 ml methanol and diluted with 0.8 ml 10 mM potassium phosphate (pH 6.5) with some precipitation. The suspension was applied to a preparative HPLC column (Whatman Magnum 20 $C_{18}$, 22 mm ID X 25 cm, 8 ml/minute. The initial mobile phase was 60/40 methanol/10 mM $K_3PO_4$, pH 6.5, and after 20 minutes the mobile phase was changed to 80/20 methanol/10 mM potassium phosphate, pH 6.5. Fractions of 8 ml each were collected, and the fractions from 31 to 33 minutes (2) were combined, diluted with water to 35% methanol, acidified with 10% HCl to pH 3, and extracted into EtOAc. The solvent was removed in vacuo and a clear slightly yellow oil identified as the titled compound was obtained.

EXAMPLE 2

Preparation of Compound A

A. Culturing MF5453

A 250 mL Erlenmeyer flask containing 54 mL of the seed medium (KF) was inoculated with MF5453 and incubated at 25°C and 85% humidity at 220 rpm for 72 hours. Ten mL of the culture was transferred to each of three 2-liter Erlenmeyer flasks containing 500 mL of the KF seed medium. These flasks were cultivated for 44 hours at 25°C, 85% humidity at 220 rpm. Seven hundred fifty mL of the resulting culture were used to inoculate each of 2 22-Liter fermentors containing 15 Liters of MBM production medium. Sterilization conditions were as follows: 122°C, 15 psi for 25 minutes. The pH of the medium after sterilization was 5.0. The fermentation conditions were as follows: 25°C, at an agitation rate of 300 rpm, and airflow rate of 4.5 liters per minute, and a back pressure of 5 psi. After 357 hours of cultivation under these conditions the batches were harvested.

B. Isolation of Compound A

Three independent fermentations (3 L), (10 L), and (10 L) were employed in the following isolation procedure. The 3 L fermentation was cultured following Example IA and each 10 L fermentation was cultured following the procedure of Example 2A. Each fermentation was filtered through celite and the mycelia extracted individually overnight (2 X) with 1 L, 3 L, and 3 L 50% methanol respectively.

The filtrates and extracts were all combined and diluted with water to 25% methanol (total volume 45 L). This was applied to a Mitsubishi HP-20 column (1.5 L) at a flow rate of 100 ml/minute. The column was then washed with water (4 L), and 40% methanol (6 L). The column was then eluted with 100% methanol (6 L).

The 100 % methanol HP-20 eluate was diluted with 10 mM $H_3PO_4$ (6 L, total volume 12 L) and extracted into $CH_2Cl_2$ (4 L). The solvent was removed in vacuo to yield 21 g of a yellow oil.

The above concentrate was dissolved in methanol (500 ml) and diluted with 20 mM $K_3PO_4$, pH 7.0 (500 ml). This solution was applied to a Dowex 1 X 2 ($Cl^-$) column (350 ml) at a rate of 25 ml/minute. The column was washed with 50/50 methanol/water (2 L), 50/50 methanol/3% NaCl solution (1 L), and the product was eluted with 90/10 methanol/30% aqueous $NH_4Cl$ solution (2 L). The methanol/$NH_4Cl$ eluate was diluted with 20 mM $H_3PO_4$ (2 L) and extracted with $CH_2Cl_2$ (2 L). The solvent was removed in vacuo to yield a thick orange oil.

A portion of the orange oil (24 mg) was dissolved in 1 ml methanol and 0.4 ml of 10 mM $H_3PO_4$ was added with some clouding . This suspension was applied to a preparative HPLC column (Whatman Magnum 20 $C_{18}$, 22 mm ID x 25 cm, 8 ml/minute, mobile phase 80/20 methanol/10 mM $H_3PO_4$ pH 2.5). The fractions eluting between 16 and 20 minutes were combined, diluted with 50 mM $H_3PO_4$ (50 ml), and extracted with $CH_2Cl_2$ (2 x 75 ml). The $CH_2Cl_2$ was removed in vacuo to yield a slightly yellowed oil. This material exhibited identical $^1H$-

NMR and UV spectra with that of Compound A isolated in Example 1.

EXAMPLE 3

As a specific embodiment of an oral composition of a compound of this invention, 20 mg of the compound from Example 1 is formulated with sufficicent finely divided lactose to provide a total amount of 580 to 590 mg to fill a size 0 hard gelatin capsule.

EXAMPLE 4

Preparation of an Ammonium Salt

A 0.1 mmol sample of the free acid of Compound (I) is dissolved in 10 ml of ethyl acetate. The resulting solution is saturated with gaseous ammonia, upon which the ammonium salt precipitates from solution.

EXAMPLE 5

Preparation of Potassium Salt

A solution of 0.1 mmol of the free acid of Compound (I) in 10 ml of methanol is treated with an aqueous or methanolic solution containing 0.3 mmol of potassium hydroxide. Evaporation of the solvent affords the tri-potassium salt. Addition of between 0.1 and 0.3 mmol of potassium hydroxide yields analogously mixtures of the mono-potassium, di-potassium and tri-potassium salts whose composition depends upon the exact amount of potassium hydroxide added.

In a similar fashion, the sodium and lithium salts of Compound (I) can be formed.

EXAMPLE 6

Preparation of a Calcium Salt

A solution of 0.1 mmol of the free acid of a compound of formula (I) in 20 ml of 6:4 methanol/water is treated with an aqueous solution of 0.1 mmol of calcium hydroxide. The solvents are evaporated to give the corresponding calcium salt.

EXAMPLE 7

Preparation of an Ethylenediamine Salt

A solution of 0.1 mmol of the free acid of a compound of formula (I) in 10 ml of methanol is treated with 0.1 mmol of ethylenediamine. Evaporation of the solvent affords the ethylenediamine salt.

The procedure can also be applied to the preparation of the N,N"-dibenzylethylenediamine salt.

EXAMPLE 8

Preparation of a Tris(hydroxymethyl)aminomethane salt

To a solution of 0.1 mmol of the free acid of a Compound of formula (I) in 10 ml of methanol is added from 0.1 to 0.3 mmol of tris(hydroxymethyl)aminomethane dissolved in 10 ml of methanol. Evaporation of the solvent gives a corresponding salt form of Compound (I) exact composition of which is determined by the molar ratio of amine added.

The method can also be applied to other amines such as, but not limited to: diethanolamine and diethylamine.

EXAMPLE 9

The preparation of a L-arginine salt

To a solution of 0.1 mmol of the free acid of a compound of formula (I) in 10 ml of 6:4 methanol/water is

treated with an aqueous solution of 0.1-0.3 mmol of L-arginine. Evaporation of the solvent affords the title salt, the exact composition of which is determined by the molar ratio of amino acid to the feee acid of Compound (I).

Similarly prepared are the salts of L-ornithine, L-lysine, and N-methylglucamine.

EXAMPLE 10

Preparation of Compound B (Method 1)

A solution of 90 mg of Compound A in 5 ml of ethyl acetate was treated with a slight excess of ethereal diazomethane. After 5 minutes excess diazomethane was removed and the solvent was evaporated to give Compound B.

EXAMPLE 11

Preparation of Compound B

A solution of 2 mg of Compound A in 0.5 ml of acetonitrile was treated at room temperature with 10 equivalents of DBU and 10 equivalents of MeI. After 2 hours the reaction was diluted with 10 ml of dichloromethane and washed successively with 10 ml of 0.1 M phosphoric acid, 10 ml of water, 10 ml of saturated sodium bicarbonate and 10 ml of water. After drying over sodium sulfate, the organic layer was concentrated and the residue was chromatographed on silica gel using mixtures of hexane and ethyl acetate to give Compound B.

The method of Example 11 is also suitable for the preparation of other ester derivatives such as 1)ethyl and another lower alkyl esters and 2)benzyl and substituted benzyl esters.

Mass Spectral Data

Mass spectra were recorded on Finnigan-MAT model MAT212 (electron impact, EI, 90 eV), MAT 90 (Fast Atom Bombardment, FAB), and TSQ70B (FAB, EI mass spectrometers. Exact mass measurements were performed at high resolution (HR-EI) using perfluorokerosene (PFK) or perfluoropolypropylene oxide (Ultramark U1600F) as internal standard. Trimethylsilyl derivatives were prepared with a 1:1 mixture of BSTFA-pyridine at room temperature.

$^{13}C$ NMR Data

The $^{13}C$ NMR spectrum of Compound A was recorded in $CD_3OD$ at 100 MHz on a Varian XL400 spectrometer. Chemical shifts are given in ppm relative to tetramethylsilane (TMS) at zero ppm using the solvent peak at 49.0 ppm as internal standard. The $^{13}C$ NMR spectrum of Compound B was recorded at 75 MHz (ambient temperature) on a Varian XL300 spectrometer in $C_6D_6$ using the solvent peak at 128.0 ppm as internal standard.

$^1H$ NMR Spectra

The $^1H$ NMR spectra were recorded at 400 MHz in $CD_3OD$ and $C_6D_6$ on a Varian XL400 spectrometer. Chemical shifts are shown in ppm relative to TMS at zero ppm using the solvent peaks at 3.30 ppm ($CD_3OD$) and 7.15 ppm ($C_6D_6$) as internal standards.

Physical Properties of the compounds of Structure I:

Compound A-the compound of structure (I) wherein $Z_1$ $Z_2$ and $Z_3$ are each hydrogen.

Mass Spectral Data:

This compound has the molecular weight 690 by FAB-MS (observed $[M+H]^+$ at m/z 691, and with addition of lithium acetate $[M{\cdot}Li_3+Li]^+$ (ie., the lithium adduct of the trilithium salt) at m/z 715). The molecular formula $C_{35}H_{46}O_{14}$ was determined by HR-EI measurement of the penta-trimethysilyl derivative (calc for $C_{35}H_{46}O_{14}+(SiC_3H_8)_5$ 1050.4864, found 1050.4829). Other critical fragment ions were observed in the silyl spectrum as follows: $[M\text{-}AcOH]^+$, calc for $C_{33}H_{44}O_{13}+(SiC_3H_8)_5$ 990.4597, found 990.4625;

$C_{23}H_{25}O_{10}$+$(SiC_3H_8)_4$, calc 749.3015, found 749.3022; [M-($CO_2H$+$SiC_3H_8$)]+, calc for $C_{34}H_{45}O_{12}$+$(SiC_3H_8)_4$ 933.4458, found 933.4450; $C_{16}H_{23}O_8$+$(SiC_3H_8)_4$, calc 631.2974, found 631.2944.
$^1$H NMR Spectrum ($CD_3OD$,40°C): See figure 1.
$^{13}$C NMR Chemical Shifts ($CD_3OD$, 40°C): 11.5, 14.3, 19.4, 20.6, 21.0, 26.7, 30.8, 33.3, 35.2, 35.6, 37.9, 41.0, 44.5, 75.8, 76.8, 80.4, 81.3, 82.7, 91.3, 106.9, 111.7, 120.0, 127.0, 129.4 (2x), 130.3(2x), 141.7, 147.9, 157.6, 166.8, 168.7, 170.3, 172.2, 172.7 ppm.
UV (MeOH) λ max 209 nm (ε = 36,000)
IR(as free acid; film on ZnSe): 3400-2500 br, 2960, 2930, 2875, 1745-1700, 1650, 1450, 1375, 1280-1225, 1185, 1135, 1020, 985, 750, and 700 $cm^{-1}$ .
Compound B-the trimethyl ester of Compound A, i.e., the compound of structure (I) wherein $Z_1$, $Z_2$ and $Z_3$ are each methyl.

Mass Spectral Data:

This compound has the molecular weight 732 by EI-MS and forms a di-(trimethylsilyl) derivative. The molecular formula $C_{38}H_{52}O_{14}$ was determined directly by HR-EI measurement (calc 732.3357, found 732.3329).
$^1$H NMR Spectrum($C_6D_6$, 22°C): See figure 2.
$^{13}$C NMR Chemical Shifts ($C_6D_6$, 75 MHz): 11.3, 14.0, 18.9, 20.2, 20.6, 26.3, 30.0, 32.1, 34.6, 34.7, 37.1, 40.2, 43.3, 51.9, 52.1, 53.3, 75.3, 76.2, 78.8, 82.0, 82.8, 89.8, 106.2, 111.8, 118.8, 126.2, 128.6(2x), 129.6(2x), 140.9, 146.6, 157.2, 166.0, 166.5, 167.2, 169.5, 170.3 ppm.

## Claims

### Claims for the following Contracting States : AT, BE, CH, DE, DK, FR, GB, IT, LI, LU, NL, SE

1. A compound of structural formula I.

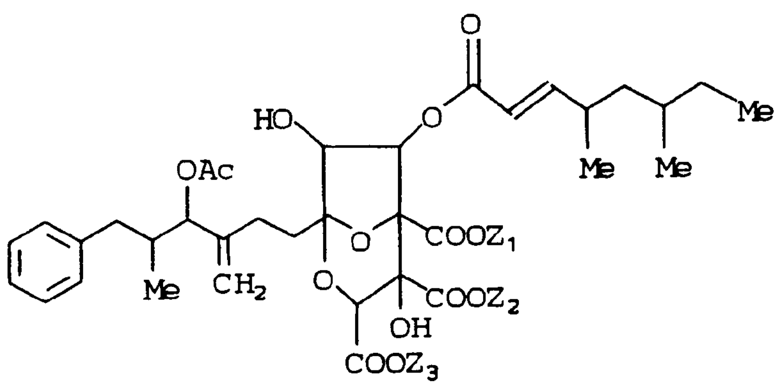

(I)

wherein
$Z_1$, $Z_2$ and $Z_3$ are each independently selected from
a) H;
b) $C_{1-5}$ alkyl;
c) $C_{1-5}$ alkyl substituted with a member of the group consisting of
i) phenyl,
ii) phenyl substituted with methyl, methoxy, halogen (Cl, Br, I, F) or hydroxy; or
a pharmaceutically acceptable salt of a compound of formula (I) in which at least one of $Z_1$, $Z_2$ and $Z_3$ is hydrogen.

2. A compound of Claim 1 wherein the relative configuration of structural formula (I) is:

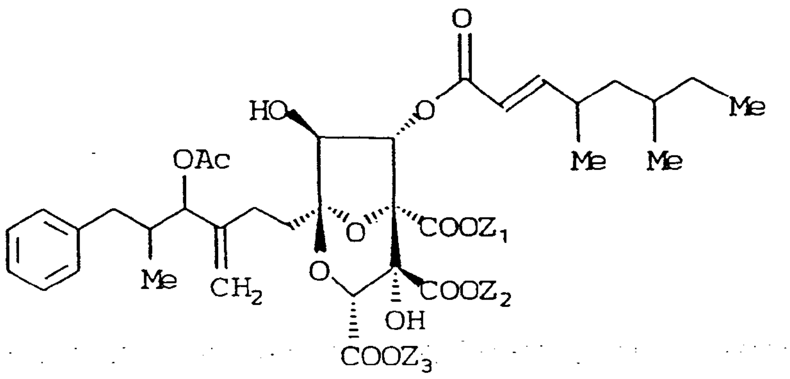

(I)

3. A compound as claimed in Claim 2 of formula:

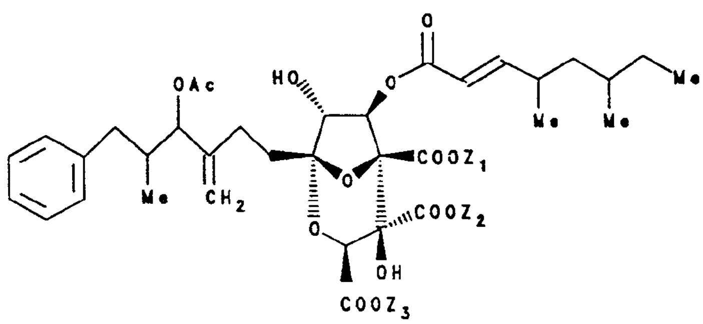

4. A compound of Claim 2 or Claim 3 in which $Z_1$, $Z_2$ and $Z_3$ are each hydrogen or a pharmaceutically acceptable salt thereof.

5. A compound of Claim 2 or Claim 3 in which $Z_1$, $Z_2$ and $Z_3$ are each methyl.

6. A compound as claimed in Claim 1 which has the following spectral data:
   $^{13}C$ NMR Chemical Shifts ($CD_3OD$, 40°C): 11.5, 14.3, 19.4, 20.6, 21.0, 26.7, 30.8, 33.3, 35.2, 35.6, 37.9, 41.0, 44.5, 75.8, 76.8, 80.4, 81.3, 82.7, 91.3, 106.9, 111.7, 120.0, 127.0, 129.4 (2x), 130.3(2x), 141.7, 147.9, 157.6, 166.8, 168.7, 170.3, 172.2, 172.7 ppm.
   UV(MeOH) λ max 209 nm (ε= 36,000)
   IR(as free acid; film on ZnSe): 3400-2500 br, 2960, 2930, 2875, 174-5-1700, 1650, 1450, 1375, 1280-1225, 1185, 1135, 1020, 985, 750, and 700 $cm^{-1}$.

7. A compound as claimed in Claim 1 which has the following spectral data:
   $^{13}C$ NMR Chemical Shifts $C_6D_6$, ($C_6D_6$, 75 MHz): 11.3, 14.0, 18.9, 20.2, 20.6, 26.3, 30.0, 32.1, 34.6, 34.7, 37.1, 40.2, 43.3, 51.9, 52.1, 53.3, 75.3, 76.2, 78.8, 82.0, 82.8, 89.8, 106.2, 111.8, 118.8, 126.2, 128.6(2x), 129.6(2x), 140.9, 146.6, 157.2, 166,0, 166.5, 167.2, 169.5, 170.3 ppm.

8. A pharmaceutical composition comprising a nontoxic therapeutically effective amount of a compound of any preceding claim and a pharmaceutically acceptable carrier.

9. A culture of MF5453 (ATCC 20986) or an active mutant thereof, capable of producing a compound of structure:

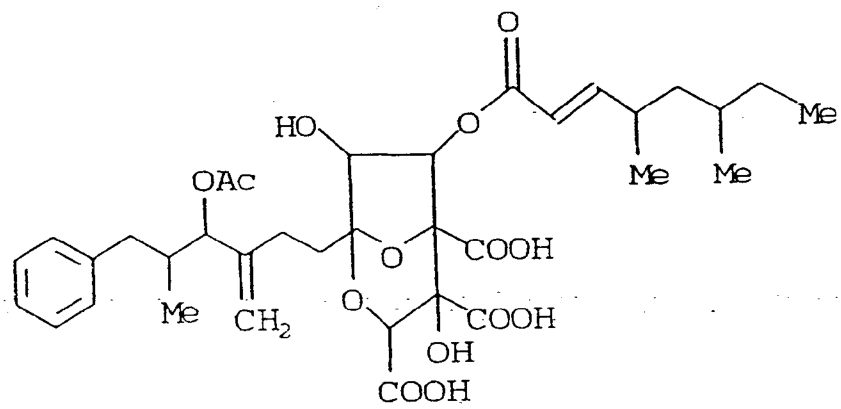

in recoverable amounts.

10. A process of making a compound of structure:

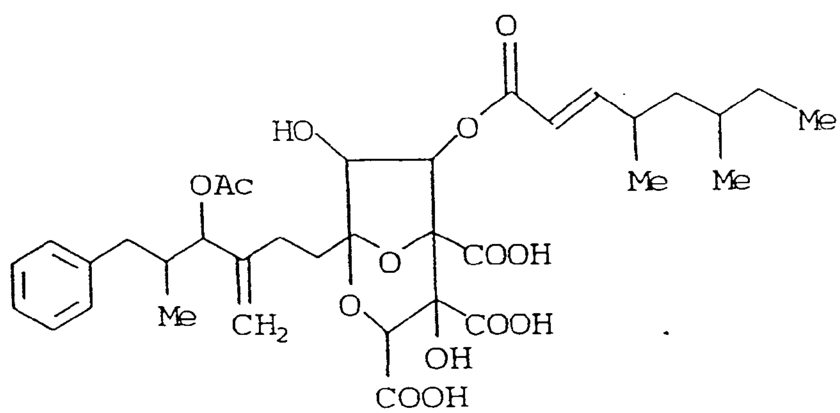

comprising cultivating MF5453 (ATCC 20986), or an active mutant thereof, in an aqueous nutrient medium containing sources of assimilable nitrogen and carbon and recovering the compound.

11. A method for inhibiting fungal growth in soil or plant parts, or in inanimate objects, comprising applying to the area where growth is to be controlled an antifungally effective amount of a compound as claimed in any of Claims 1 to 7.

12. The use of a compound as claimed in any of Claims 1 to 7 for the manufacture of a medicament suitable for inhibiting fungal growth in a living organism.

13. The use of a compound as claimed in any of Claims 1 to 7 for the manufacture of a medicament for treating hypercholesterolemia.

**Claims for the following Contracting States : ES, GR**

1. A process for producing the compound of formula I

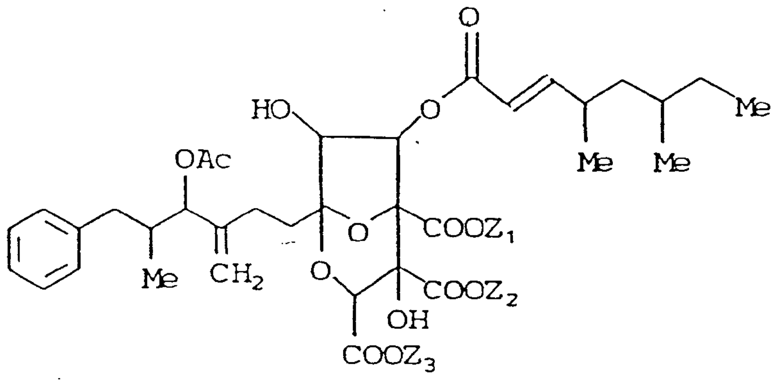

(I)

wherein

$Z_1$, $Z_2$ and $Z_3$ are each independently selected from

a) H;

b) $C_{1-5}$ alkyl;

c) $C_{1-5}$ alkyl substituted with a member of the group consisting of

i) phenyl,

ii) phenyl substituted with methyl, methoxy, halogen (Cl, Br, I, F) or hydroxy; or

a pharmaceutically acceptable salt of a compound of formula (I) in which at least one of $Z_1$, $Z_2$ and $Z_3$ is hydrogen,

which comprises cultivating MF5453(ATCC 20986) or an active mutant thereof, in an aqueous nutrient medium containing sources of assimilable carbon and nitrogen, and, if desired, forming a pharmaceutically acceptable salt.

2. A process for producing a compound of claim 1 wherein the relative configuration of structural formula (I) is:

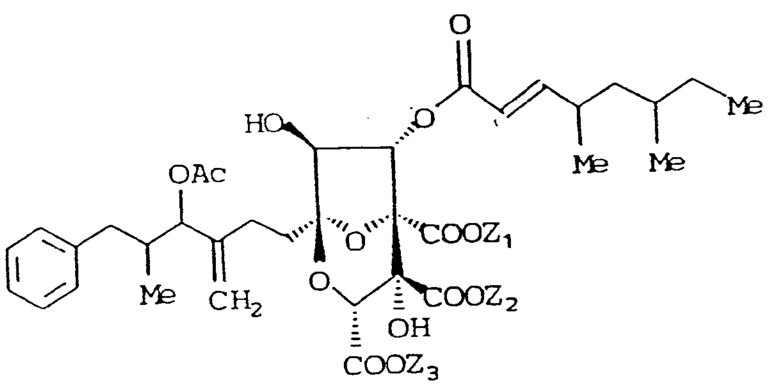

(I)

3. A process for producing a compound of claim 2 in which $Z_1$, $Z_2$ and $Z_3$ are each hydrogen or a pharmaceutically acceptable salt thereof.

4. A process for producing a compound of claim 2 in which $Z_1$, $Z_2$ and $Z_3$ are each methyl.

5. A method for inhibiting fungal growth in soil or plant parts, or in inanimate objects, comprising applying to the area where growth is to be controlled an antifungally effective amount of a compound as claimed in claim 1.

6. A process as claimed in claim 5 wherein the conventional means comprise treatment with methyl iodide

or diazomethane.

7. A process as claimed in claim 5 or claim 6 for the preparation of a compound of formula (I) wherein $Z_1$, $Z_2$ and $Z_3$ are each methyl.

8. A process as claimed in claim 1 for the preparation of a compound which has the following spectral data:
$^{13}$C NMR Chemical Shifts ($CD_3OD$, 40°C) : 11.5, 14.3, 19.4, 20.6, 21.0, 26.7, 30.8, 33.3, 35.2, 35.6, 37.9, 41.0, 44.5, 75.8, 76.8, 80.4, 81.3, 82.7, 91.3, 106.9, 111.7, 120.0, 127.0, 129.4 (2x), 130.3(2x), 141.7, 147.9, 157.6, 166.8, 168.7, 170.3, 172.2, 172.7 ppm.
UV(MeOH) $\lambda$ max 209 nm ($\varepsilon$ = 36,000)
IR(as free acid; film on ZnSe): 3400-2500 br, 2960, 2930, 2875, 1745-1700, 1650, 1450, 1375, 1280-1225, 1185, 1135, 1020, 985, 750, and 700 $cm^{-1}$.

9. A process as claimed in claim 1 for the preparation of a compound which has the following spectral data:
$^{13}$C NMR Chemical Shifts ($C_6D_6$, 75 MHz): 11.3, 14.0, 18.9, 20.2, 20.6, 26.3, 30.0, 32.1, 34.6, 34.7, 37.1, 40.2, 43.3, 51.9, 52.1, 53.3, 75.3, 76.2, 78.8, 82.0, 82.8, 89.8, 106.2, 111.8, 118.8, 126.2, 128.6(2x), 129.6(2x), 140.9, 146.6, 157.2, 166.0, 166.5, 167.2, 169.5, 170.3 ppm.

10. A process as claimed in any preceding claim wherein the cultivation is under aerobic conditions.

11. A process as claimed in any preceding claim which comprises inoculating spores or mycelia of MF5453 (ATCC 20986) or an active mutant thereof, into an aqueous nutrient medium containing sources of assimilable carbon and nitrogen, and, if desired, forming a pharmaceutically acceptable salt.

12. A method for inhibiting fungal growth in soil or plant parts, or in inanimate objects, comprising applying to the area where growth is to be controlled an antifungally effective amount of a compound of formula (I).

## Patentansprüche

### Patentansprüche für folgende Vertragsstaaten : AT, BE, CH, DE, DK, FR, GB, IT, LI, LU, NL, SE

1. Verbindung der Strukturformel I

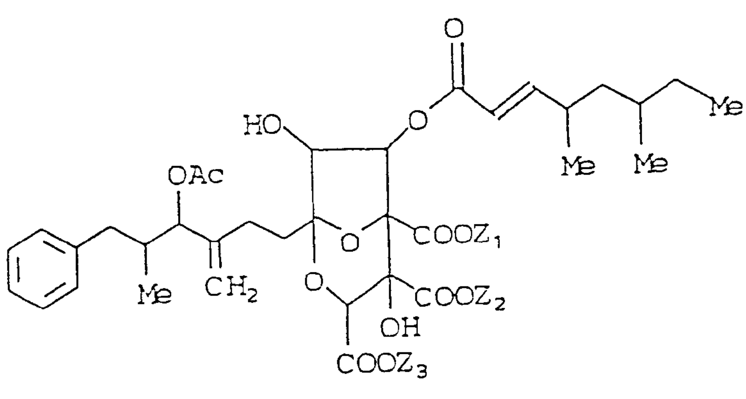

(I)

wobei
$Z_1$, $Z_2$, und $Z_3$ jeweils unabhängig voneinander ausgewählt sind aus
a) H;
b) $C_1$-$C_5$-Alkyl;
c) $C_1$-$C_5$-Alkyl, substituiert mit einen Substituenten der Gruppe
i) Phenyl,
ii) Phenyl, substituiert mit Methyl, Methoxy, Halogen (Cl, Br, I, F) oder Hydroxy, oder
ein pharmazeutisch annehmbares Salz einer Verbindung der Formel (I), in der mindestens einer der Reste $Z_1$, $Z_2$, und $Z_3$ Wasserstoff ist.

2. Verbindung nach Anspruch 1, wobei die relative Konfiguration der Strukturformel (I)

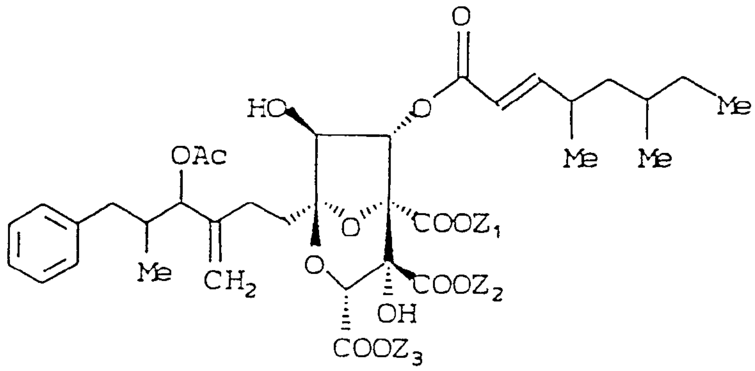

(I)

ist.

3. Verbindung nach Anspruch 2, der Formel

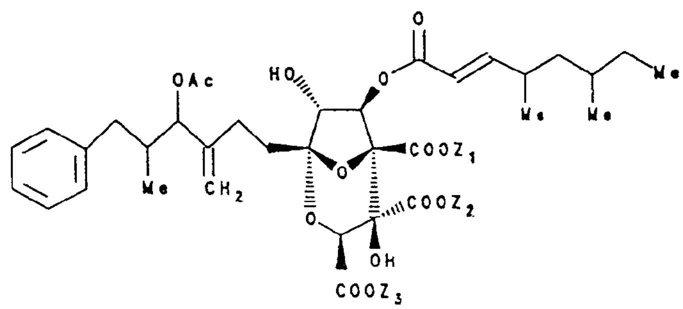

4. Verbindung nach Anspruch 2 oder 3, in der $Z_1$, $Z_2$, und $Z_3$ jeweils Wasserstoff bedeuten, oder ein pharmazeutisch annehmbares Salz davon.

5. Verbindung nach Anspruch 2 oder 3, in der $Z_1$, $Z_2$, und $Z_3$ jeweils Methyl bedeuten.

6. Verbindung nach Anspruch 1 mit den folgenden Spektral-Daten:
<u>$^{13}C$ NMR, chemische Verschiebungen ($CD_3OD$, 40°C):</u> 11,5, 14,3, 19,4, 20,6, 21,0, 26,7, 30,8, 33,3, 35,2, 35,6, 37,9, 41,0, 44,5, 75,8, 76,8, 80,4, 81,3, 82,7, 91,3, 106,9, 111,7, 120,0, 127,0, 129,4(2x), 130,3(2x) , 141,7, 147,9, 157,6, 166,8, 168,7, 170,3, 172,2, 172,7 ppm;
<u>UV (MeOH)</u> $\lambda_{max}$ 209 nm ($\varepsilon$ = 36 000)
<u>IR (als freie Säure; Film auf ZnSe):</u> 3400-2500 br, 2960, 2930, 2875, 1745-1700, 1650, 1450, 1375, 1280-1225, 1185, 1135, 1020, 985, 750 und 700 $cm^{-1}$.

7. Verbindung nach Anspruch 1 mit den folgenden Spektral-Daten:
<u>$^{13}C$ NMR, chemische Verschiebungen ($C_6D_6$, 75 MHz) :</u> 11,3, 14,0, 18,9, 20,2, 20,6, 26,3, 30,0, 32,1, 34,6, 34,7, 37,1, 40,2, 43,3, 51,9, 52,1, 53,3, 75,3, 76,2, 78,8, 82,0, 82,8, 89,8, 106,2, 111,8, 118,8, 126,2, 128,6 (2x), 129,6 (2x) , 140,9, 146,6, 157,2, 166,0, 166,5, 167,2, 169,5, 170,3 ppm.

8. Pharmazeutisches Mittel, umfassend eine nicht-toxische wirksame Menge einer Verbindung nach einem der vorangehenden Ansprüche und einen pharmazeutisch annehmbaren Träger.

9. Kultur von MF5453 (ATCC 20986) oder einer aktiven Mutante davon, die in der Lage ist, eine Verbindung der Struktur

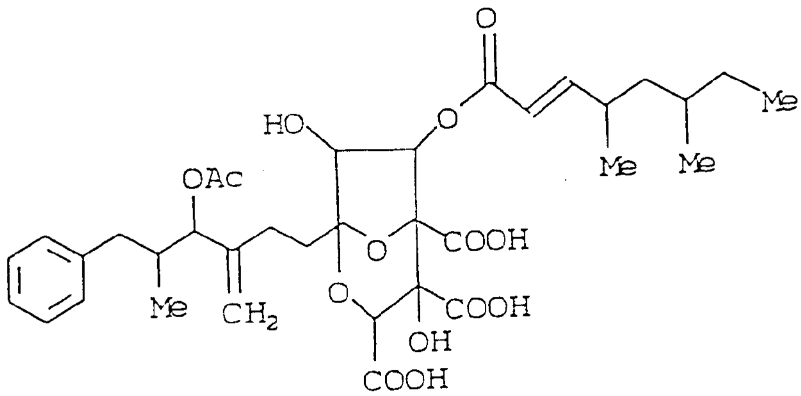

in gewinnbaren Mengen zu bilden.

**10.** Verfahren zur Herstellung einer Struktur

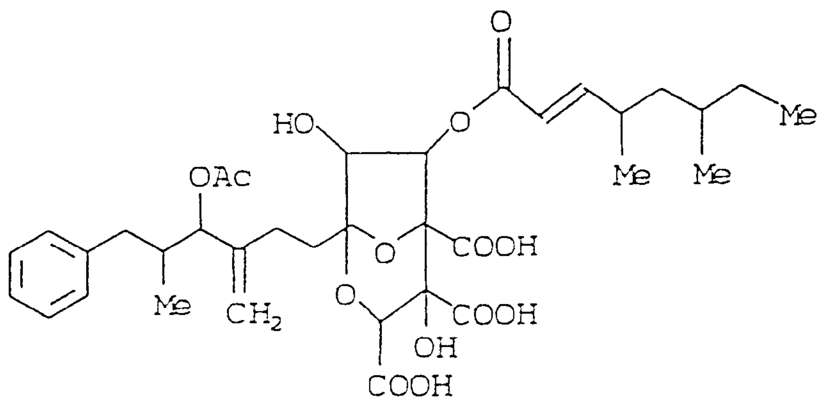

umfassend die Züchtung von MF5453 (ATCC 20986) oder einer aktiven Mutante davon, in einem wäßrigen Nährmedium, enthaltend Quellen für assimilierbaren Stickstoff und Kohlenstoff, und Gewinnung der Verbindung.

**11.** Verfahren zur Hemmung des Pilzwachstums im Boden oder in Pflanzenteilen oder in unbelebten Objekten, umfassend das Aufbringen einer gegen Pilze wirksamen Menge einer Verbindung nach einem der Ansprüche 1 bis 7 auf den Bereich, auf dem das Wachstum kontrolliert werden soll.

**12.** Verwendung einer Verbindung nach einem der Ansprüche 1 bis 7 zur Herstellung eines Arzneimittels zur Hemmung des Pilzwachstums in einem lebenden Organismus.

**13.** Verwendung einer Verbindung nach einem der Ansprüche 1 bis 7 zur Herstellung eines Arzneimittels zur Behandlung von Hypercholesterinämie.

**Patentansprüche für folgende Vertragsstaaten : ES, GR**

**1.** Verfahren zur Herstellung einer Verbindung der Formel I

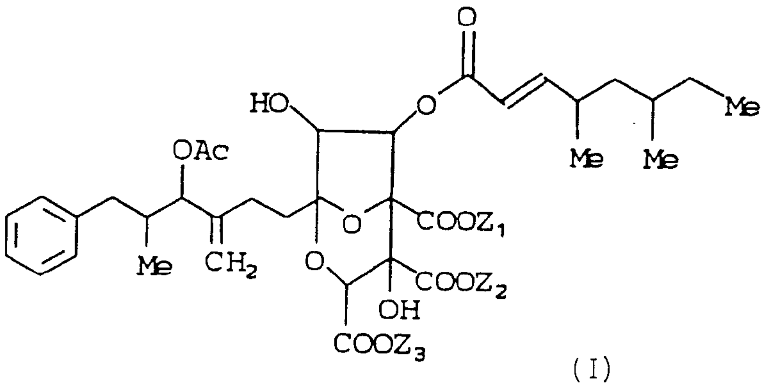

(I)

wobei
$Z_1$, $Z_2$, und $Z_3$ jeweils unabhängig voneinander ausgewählt sind aus
a) H;
b) $C_1$-$C_5$-Alkyl;
c) $C_1$-$C_5$-Alkyl, substituiert mit einem Substituenten der Gruppe
i) Phenyl,
ii) Phenyl, substituiert mit Methyl, Methoxy, Halogen (Cl, Br, I, F) oder Hydroxy, oder
eines pharmazeutisch annehmbaren Salzes einer Verbindung der Formel (I), in der mindestens einer der Reste $Z_1$, $Z_2$, und $Z_3$ Wasserstoff ist, umfassend die Züchtung von MF5453 (ATCC 20986) oder einer aktiven Mutante davon, in einem wäßrigen Nährmedium, enthaltend Quellen für assimilierbaren Kohlenstoff und Stickstoff, und, soweit erwünscht, Bildung eines pharmazeutisch annehmbaren Salzes.

2. Verfahren zur Herstellung einer Verbindung nach Anspruch 1, wobei die relative Konfiguration der Strukturformel (I)

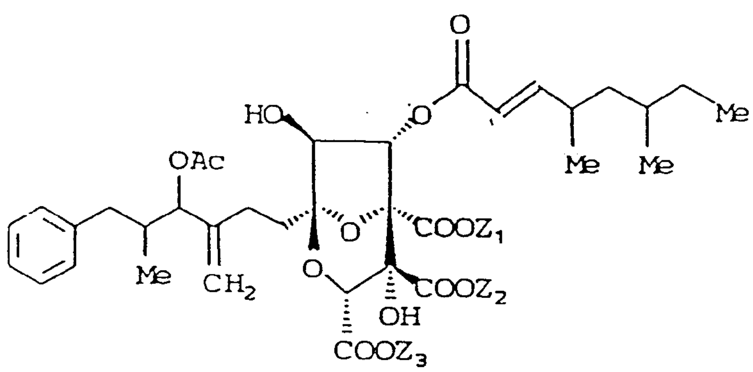

(I)

ist.

3. Verfahren zur Herstellung einer Verbindung nach Anspruch 2, in der $Z_1$, $Z_2$, und $Z_3$ jeweils Wasserstoff bedeuten, oder eines pharmazeutisch annehmbaren Salzes davon.

4. Verfahren zur Herstellung einer Verbindung nach Anspruch 2, in der $Z_1$, $Z_2$, und $Z_3$ jeweils Methyl bedeuten.

5. Verfahren zur Hemmung des Pilzwachstums im Boden oder in Pflanzenteilen oder in unbelebten Objekten, umfassend das Aufbringen einer gegen Pilze wirksamen Menge einer Verbindung nach Anspruch 1 auf den Bereich, auf dem das Wachstum kontrolliert werden soll.

6. Verfahren nach Anspruch 5, wobei die üblichen Maßnahmen eine Behandlung mit Methyliodid oder Diaziomethan umfassen.

7. Verfahren nach Anspruch 5 oder Anspruch 6 zur Herstellung einer Verbindung der Formel (I), in der $Z_1$,

$Z_2$, und $Z_3$ jeweils Methyl bedeuten.

**8.** Verfahren nach Anspruch 1 zur Herstellung einer Verbindung mit den folgenden Spektral-Daten:
$^{13}C$ NMR chemische Verschiebungen ($CD_3OD$, 40°C) : 11,5, 14,3, 19,4, 20,6, 21,0, 26,7, 30,8, 33,3, 35,2, 35,6, 37,9, 41,0, 44,5, 75,8, 76,8, 80,4, 81,3, 82,7, 91,3, 106,9, 111,7, 120,0, 127,0, 129,4 (2x), 130,3 (2x), 141,7, 147,9, 157,6, 166,8, 168,7, 170,3, 172,2, 172,7 ppm;
UV (MeOH): $\lambda_{max}$ 209 nm ($\varepsilon$ = 36 000)
IR (als freie Säure; Film auf ZnSe): 3400-2500 br, 2960, 2930, 2875, 1745-1700, 1650, 1450, 1375, 1280-1225, 1185, 1135, 1020, 985, 750 und 700 $cm^{-1}$.

**9.** Verfahren nach Anspruch 1 zur Herstellung einer Verbindung mit den folgenden Spektral-Daten:
$^{13}C$ NMR chemische Verschiebungen ($C_6D_6$, 75 MHz) : 11,3, 14,0, 18,9, 20,2, 20,6, 26,3, 30,3, 32,1, 34,6, 34,7, 37,1, 40,2, 43,3, 51,9, 52,1, 53,3, 75,3, 76,2, 78,8, 82,0, 82,8, 89,8, 106,2, 111,8, 118,8, 126,2, 128,6 (2x), 129,6 (2x), 140,9, 146,6, 157,2, 166,0, 166,5, 167,2, 169,5, 170,3 ppm.

**10.** Verfahren nach einem der vorangehende Ansprüche, wobei die Züchtung unter aeroben Bedingungen durchgeführt wird.

**11.** Verfahren nach einem der vorangehende Ansprüche, umfassend das Einimpfen von Sporen oder Myzel von MF5453 (ATCC 20986) oder einer aktiven Mutante davon in ein wäßriges Nährmedium, enthaltend Quellen für assimilierbaren Kohlenstoff und Stickstoff, und, soweit erwünscht, Bildung eines pharmazeutisch annehmbaren Salzes.

**12.** Verfahren zur Hemmung des Pilzwachstums im Boden oder in Pflanzenteilen oder in unbelebten Objekten, umfassend das Aufbringen einer gegen Pilze wirksamen Menge einer Verbindung der Formel (I) auf den Bereich, auf dem das Wachstum kontrolliert werden soll.

## Revendications

### Revendications pour les Etats contractants suivants : AT, BE, CH, DE, DK, FR, GB, IT, LI, LU, NL, SE

**1.** Composé de formule structurelle I

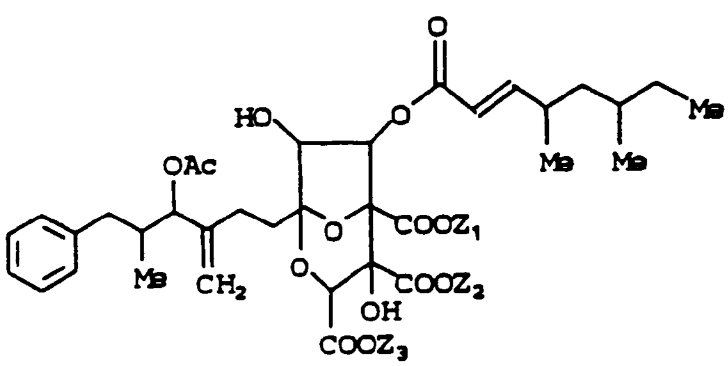

(I)

dans lequel
$Z_1$, $Z_2$ et $Z_3$ sont chacun indépendamment choisis parmi:
a) H;
b) un alkyle en $C_1$ en $C_5$
c) un alkyle en $C_1$ à $C_5$ substitué avec un élément du groupe formé par:
i) un phényle
ii) un phényle substitué avec un groupe méthyle, méthoxy, un atome d'halogène (Cl, Br, I, F) ou un groupe hydroxy; ou

un sel acceptable sur le plan pharmaceutique d'un composé de formule (I) dans lequel au moins un de $Z_1$, $Z_2$ et $Z_3$ représente un atome d'hydrogène.

2. Composé selon la revendication 1, dans lequel la configuration relative de la formule structurelle (I) est

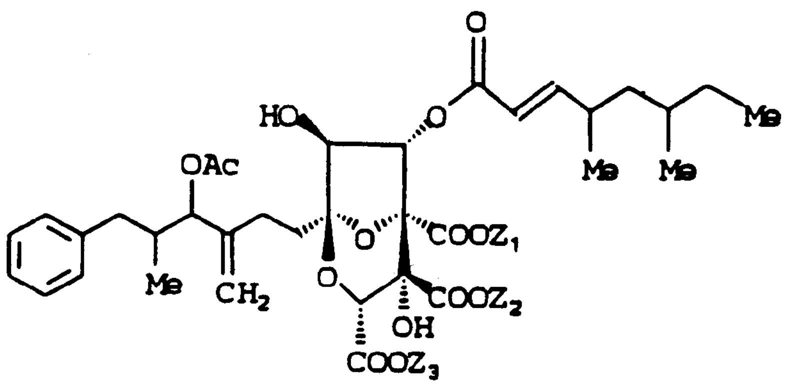

(I)

3. Composé selon la revendication 2 de formule

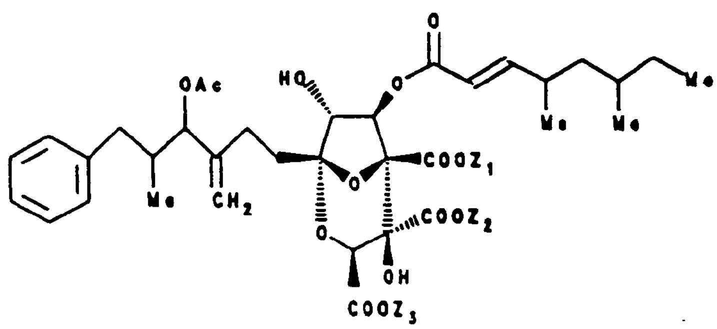

4. Composé selon la revendication 2 ou 3, dans lequel $Z_1$, $Z_2$ et $Z_3$ sont chacun un atome d'hydrogène ou un sel de celui-ci acceptable sur le plan pharmaceutique.

5. Composé selon la revendication 2 ou 3, dans lequel $Z_1$, $Z_2$ et $Z_3$ sont chacun un groupe méthyle.

6. Composé selon la revendication 1, qui a les données spectrales suivantes:
décalages chimiques RMN $^{13}D$ ($CD_3OD$, 40°C):
11,5; 14,3; 19,4; 20,6; 21,0; 26,7; 30,8; 33,3; 35,2; 35,6; 37,9; 41,0; 44,5; 75,8; 76,8; 80,4; 81,3; 82,7; 91,3; 106,9; 111,7; 120,0; 127,0; 129,4 (2x); 130,3 (2x); 141,7; 147,9, 157,6, 166,8, 168,7; 170,3, 172,2; 172,7 ppm.
UV(MeOH) $\lambda$ max 209 nm ($\varepsilon$ = 36 000)
IR (sous forme d'acide libre: film sur ZnSe) : 3400 à 2500 br, 2960, 2930, 2875, 1745 à 1700, 1650, 1450, 1375, 1280 à 1225, 1185, 1135, 1020, 985, 750 et 700 $cm^{-1}$.

7. Composé selon la revendication 1 qui a les données spectrales suivantes:
décalages chimiques RMN $^{13}C$($C_6D_6$, 75 MHz); 11,3; 14,0; 18,9; 20,2; 20,6; 26,3; 30,0; 32,1; 34,6; 34,7; 37,1; 40,2; 43,3; 51,9; 52,1; 53,3; 75,3; 76,2; 78,8; 82,0; 82,8; 89,8; 106,2; 111,8; 118,8; 126,2; 128,6 (2x); 129,6 (2x); 140,9; 146,6; 157,2; 166,0; 166,5; 167,2; 169,5; 170,3 ppm.

8. Composition pharmaceutique comprenant une quantité non-toxique efficace sur le plan thérapeutique d'un composé selon l'une quelconque des revendications précédentes et une charge acceptable sur le plan pharmaceutique.

9. Culture de MF5453 (ATCC 20986) ou d'un mutant actif de celui-ci, capable de produire un composé de

structure

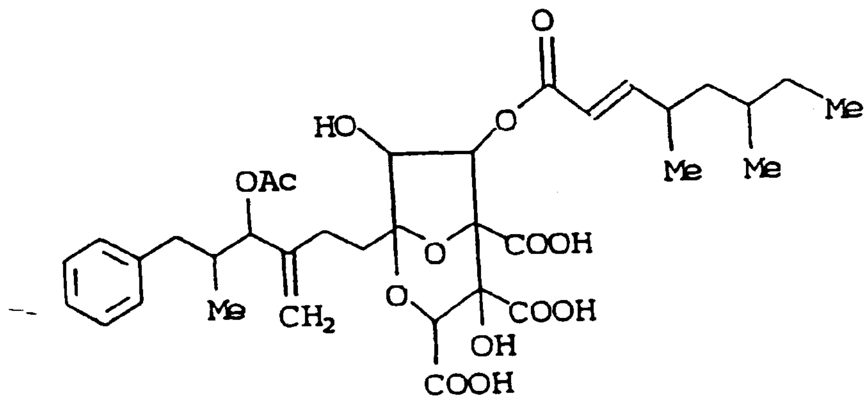

en des quantités récupérables.

**10.** Procédé pour fabriquer un composé de structure

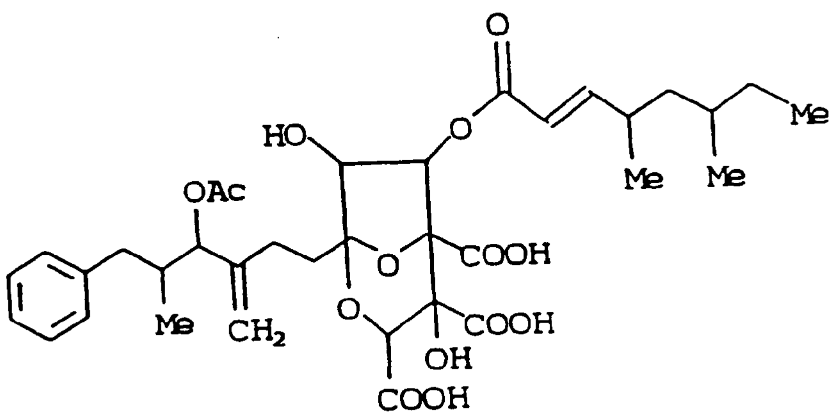

comprenant la culture de MF5453 (ATTC 20 986), ou un mutant actif de celui-ci, dans un milieu nutritif aqueux contenant des sources d'azote et de carbone assimilables et la récupération du composé.

**11.** Procédé pour inhiber la croissance fongique dans le sol ou des parties de plantes, ou dans des objets inanimés, comprenant l'application à la zone, où l'on doit lutter contre la croissance, d'une quantité efficace sur le plan antifongique d'un composé selon l'une quelconque des revendications 1 à 7.

**12.** Utilisation d'un composé selon l'une quelconque des 1 à 7, pour la fabrication d'un médicament approprié pour inhiber la croissance fongique dans un organisme vivant.

**13.** Utilisation d'un composé selon l'une quelconque des revendications 1 à 7, pour la fabrication d'un médicament destiné au traitement de l'hypercholestérolémie.

**Revendications pour les Etats contractants suivants : ES, GR**

**1.** Procédé pour produire le composé de formule I

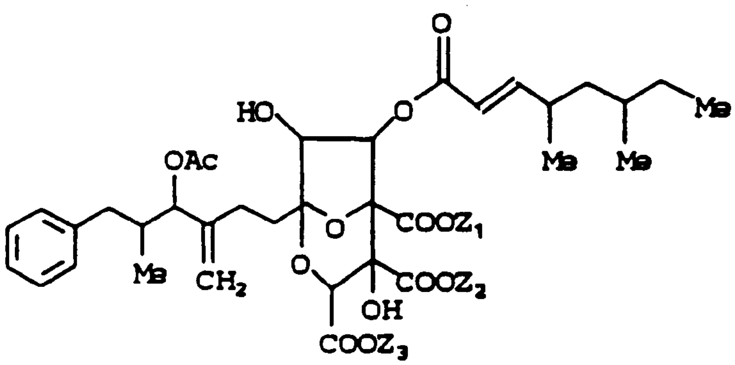

(I)

dans lequel

$Z_1$, $Z_2$ et $Z_3$ sont chacun indépendamment choisis parmi:

a) H;

b) un alkyle en $C_1$ en $C_5$

c) un alkyle en $C_1$ à $C_5$ substitué avec un élément du groupe formé par:

i) un phényle

ii) un phényle substitué avec un groupe méthyle, méthoxy, un atome d'halogène (Cl, Br, I, F) ou un groupe hydroxy; ou

un sel acceptable sur le plan pharmaceutique d'un composé de formule (I) dans lequel au moins un de $Z_1$, $Z_2$ et $Z_3$ représente un atome d'hydrogène qui comprend la culture de MF5453 (ATCC 20986) ou d'un mutant actif de celui-ci, dans un milieu nutritif aqueux contenant des sources d'azote et de carbone assimilable et, si on le souhaite, la formation d'un sel pharmaceutiquement acceptable.

**2.** Procédé pour produire un composé selon la revendication 1, dans lequel la configuration relative de la formule structurelle (I) est

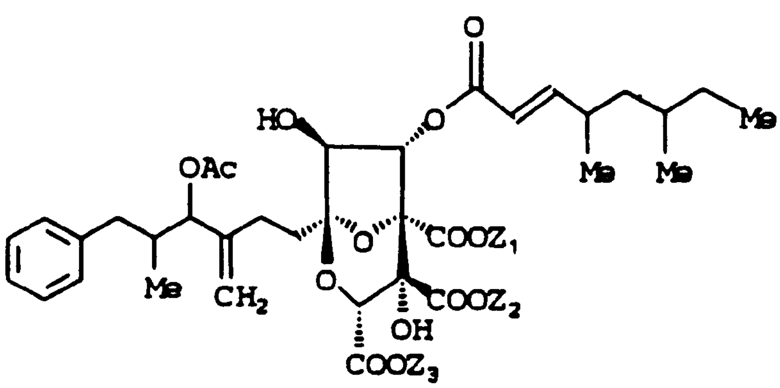

(I)

**3.** Procédé pour produire un composé selon la revendication 2, dans lequel $Z_1$, $Z_2$ et $Z_3$ sont chacun un atome d'hydrogène ou un sel de celui-ci acceptable sur le plan pharmaceutique.

**4.** Procédé pour produire un composé selon la revendication 2, dans lequel $Z_1$, $Z_2$ et $Z_3$ sont chacun un groupe méthyle.

**5.** Procédé pour inhiber la croissance fongique dans le sol ou dans des parties de plantes, ou dans des objets inanimés, comprenant l'application dans la zone, où l'on doit lutter contre la croissance, d'une quantité efficace sur le plan antifongique d'un composé selon la revendication 1.

**6.** Procédé selon la revendication 5, dans lequel le moyen classique comprend un traitement avec de l'iodure

de méthyle ou du diazométhane.

7. Procédé selon la revendication 5, ou la revendication 6 pour la préparation d'un composé de formule (I) dans lequel $Z_1$, $Z_2$, et $Z_3$ sont chacun un groupe méthyle.

8. Procédé selon la revendication 1 pour la préparation d'un composé qui a les données spectrales suivantes:
décalages chimiques RMN $^{13}C$ ($CD_3OD$, 40°C):
11,5; 14,3; 19,4; 20,6; 21,0; 26,7; 30,8; 33,3; 35,2; 35,6; 37,9; 41,0; 44,5; 75,8; 76,8; 80,4; 81,3; 82,7; 91,3; 106,9; 111,7; 120,0; 127,0; 129,4 (2x); 130,3 (2x); 141,7; 147,9, 157,6, 166,8, 168,7; 170,3, 172,2; 172,7 ppm.
UV(MeOH) λ max 209 nm (ε = 36 000)
IR (sous forme d'acide libre: film sur ZnSe): 3400 à 2500 br, 2960, 2930, 2875, 1745 à 1700, 1650, 1450, 1375, 1280 à 1225, 1185, 1135, 1020, 985, 750 et 700 $cm^{-1}$.

9. Procédé selon la revendication 1 pour la préparation d'un composé qui a les données spectrales suivantes Décalages chimiques RMN $^{13}C$ ($C_6D_6$, 75 MHz); 11,3; 14,0; 18,9: 20,2; 20,6; 26,3; 30,0; 32,1; 34,6; 34,7; 37,1; 40,2; 43,3; 51,9; 52,1; 53,3; 75,3; 76,2; 78,8; 82,0; 82,8; 89,8; 106,2; 111,8; 118,8; 126,2; 128,6 (2x); 129,6 (2x); 140,9; 146,6; 157,2; 166,0; 166,5; 167,2; 169,5; 170,3 ppm.

10. Procédé selon l'une quelconque des revendications précédentes dans laquelle la culture se fait dans des conditions aérobies.

11. Procédé selon l'une quelconque des revendications précédentes, qui comprend l'inoculation de spores ou de mycélium de MF5453 (ATTC 20 986) ou d'un mutant actif de celui-ci, dans un milieu aqueux nutritif contenant des sources de carbone et d'azote assimilables, et, si on le souhaite, la formation d'un sel acceptable sur le plan pharmaceutique.

12. Procédé pour inhiber la croissance fongique dans le sol ou dans des parties de plantes, ou dans les objets inanimés, comprenant l'application à la zone, où on doit lutter contre la croissance, d'une quantité efficace sur le plan antifongique d'un composé de formule (I).

FIGURE 1

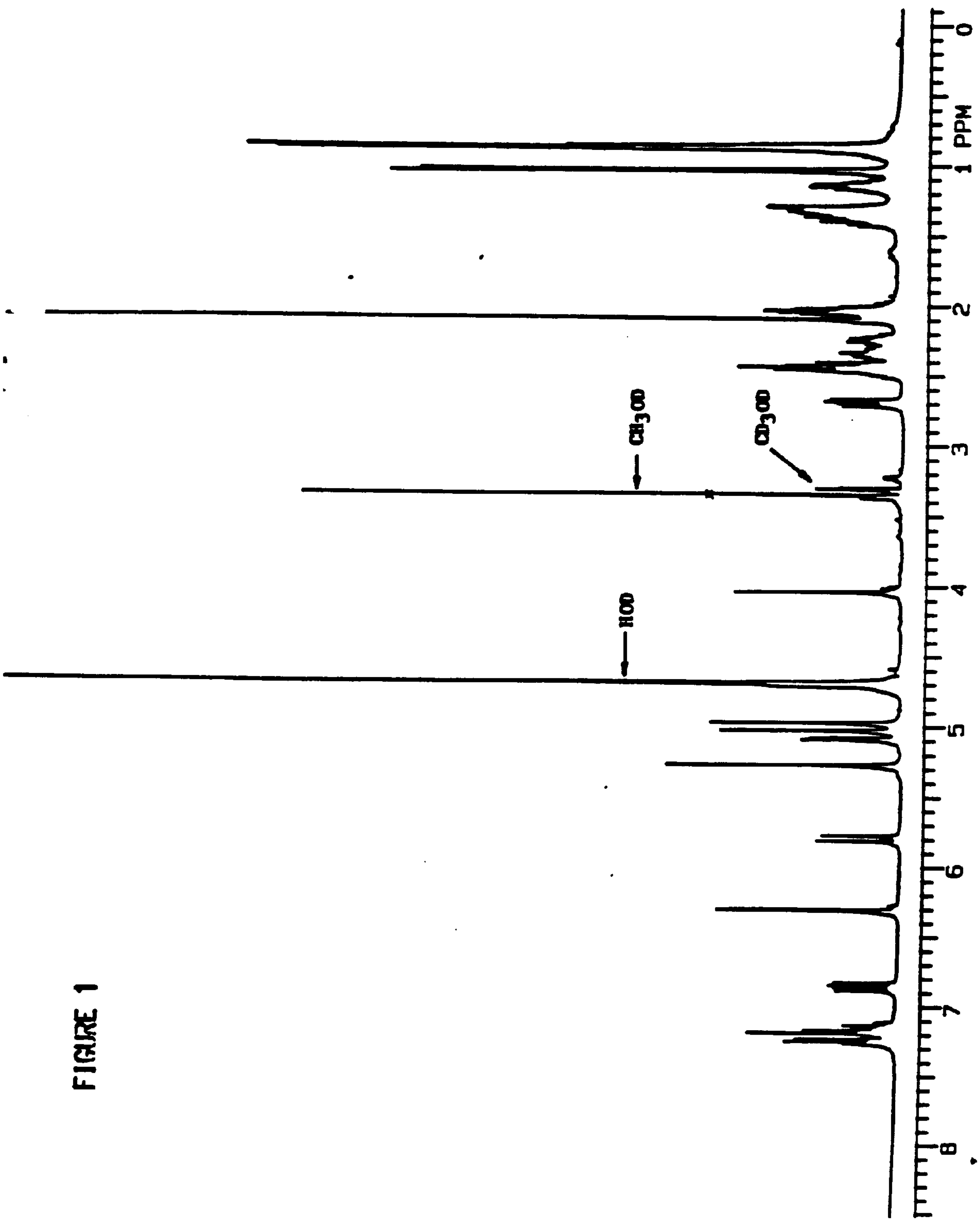

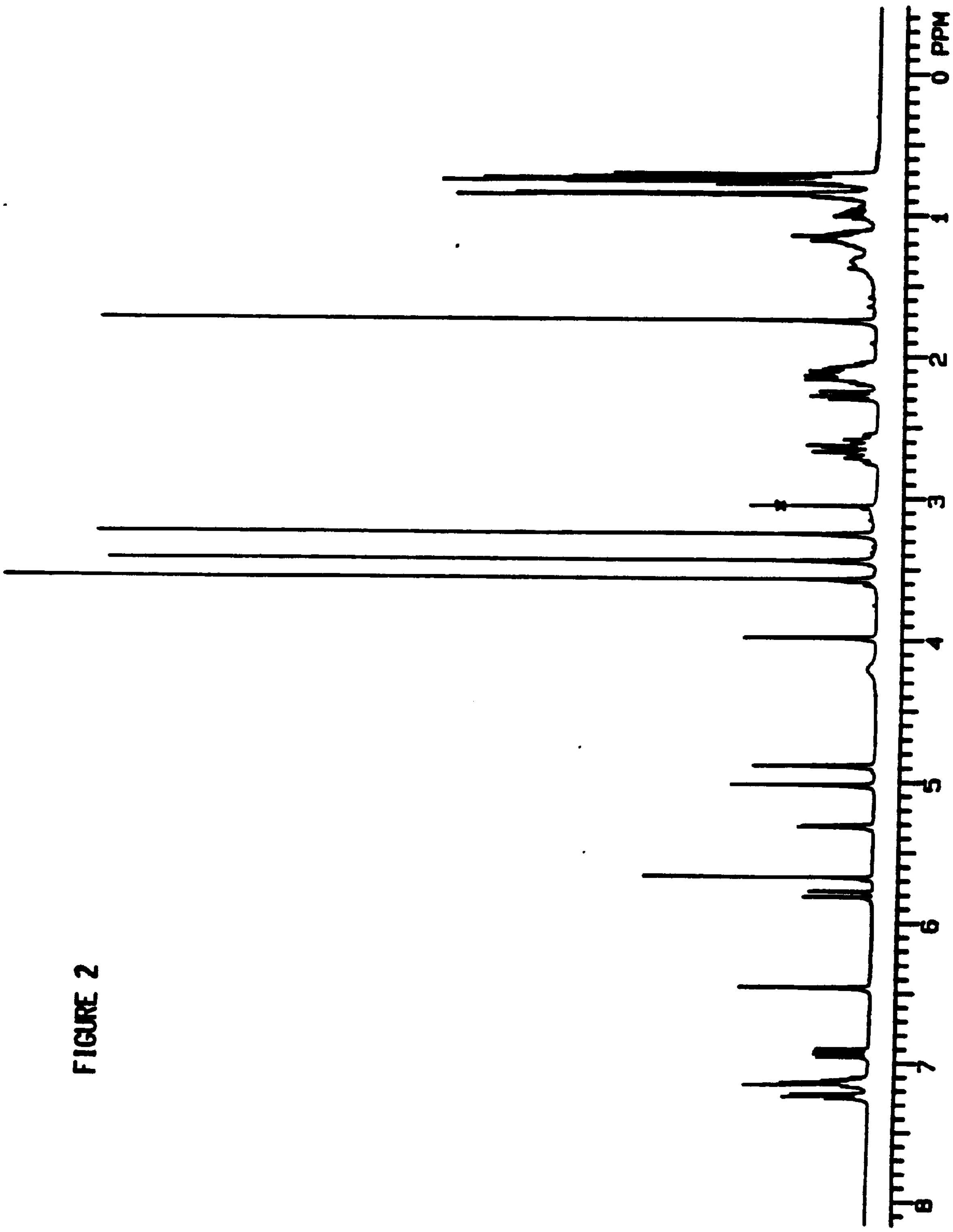


FIGURE 2